# EUROPEAN PATENT SPECIFICATION

(11) **EP 1 645 252 B1**
(45) Date of publication and mention of the grant of the patent: **15.02.2012**
(21) Application number: 04738219.7
(22) Date of filing: 09.06.2004
(51) Int. Cl.: A61F 5/56

(54) **REMOVABLE INTRA-ORAL CORRECTIVE ANTI-SNORING DEVICE**
ENTFERNBARE INTRAORALE KORRIGIERENDE ANTI-SCHNARCH-VORRICHTUNG
DISPOSITIF CORRECTEUR ANTI-RONFLEMENT INTRA-ORAL AMOVIBLE

(30) Priority: 09.06.2003 CN 03129136
(43) Date of publication of application: 12.04.2006
(73) Proprietor: Shanghai Guang Ren Anti-Snoring Health Center, Huangpu District Shanghai 200010 (CN)
(72) Inventor: Chen, Haidong, Shanghai 200081 (CN); CHEN, Yueyang, Shanghai 200081 (CN)
(74) Representative: Adorno, Silvano
(86) International application number: PCT/CN2004/000621
(87) International publication number: WO 2004/108028

(56) References cited:
- EP-A1- 0 679 378
- EP-A1- 0 679 378
- WO-A-96/11653
- CN-U- 2 075 076
- DE-A1- 10 011 687
- DE-A1- 19 512 761
- DE-U1- 20 214 663
- DE-U1- 20 214 663
- US-A- 3 132 647
- US-A- 4 669 459
- US-A- 5 915 385
- US-B1- 6 467 484

## Description

### FIELD OF THE INVENTION

The present invention relates to a snore curing field, especially relates to a comfortable type removable tongue position corrective anti-snoring & anti-suffocating device for preventing snore and remitting sleep apnoea syndrome (i.e. the so-called snoring with suffocating). The closest prior art is DE 20214663 U1, which defines the preamble of claim 1.

### BACKGROUND OF THE INVENTION

The snore is a rebarbative but very familiar physiological phenomenon, it results in not only affecting other sleeping person in the same or adjacent room, but also an apnoea and even suffocation for short of oxygen, then induces many diseases, harms to health, reduces the efficiency of work and learning and even reduces life-span. The curing method for snore includes operation, medicine and positive press breath machine assistant and orthopedics method. Among which, the orthopedics method is not traumatic, has no side-effects, is simple and convenient, thus has a well development in the future.

The Chinese utility model 90215295 discloses an orthopedics method of NiTi shape memory alloy anti-snoring device, it is made from the NiTi shape memory alloy material to manufacture a force piece that suitable to the normal tongue base or soft palate, and acts on the affected part (i.e. the big tongue base and uvula) of the patient's body to stop the snoring. It has, however, the following drawbacks in practice: (1) it often suffers from the upward pushing force from the big tongue base since it is mostly applied on lower jaw, the pushing force is conducted through collar to the teeth and produce some bad pain as pulling out teeth; (2) since it directly acts on the uvula position, moreover after research people find that the other tissue near the uvula (e.g. tongue palate bow and palate tonsil) is more sensitive and easy to cause itch and nausea; (3) since it acts directly on the big tongue base, and after research we find that the big tongue base itself is specially sensitive and easier to cause pain and nausea. All the three drawbacks above are severely affecting its application, so the patent is not practical with only 1 to 2 suitable patients from 100 patients.

Other specific applications of the orthopedics method are various oral anti-snoring devices. Before the present invention, the German patent DE 202 14 663 U1 (hereinafter named as reference D1), the U.S. Patent US-A-3 132 647 (hereinafter named as reference D2), the U.S. Patent US-B1-6 467 484 (hereinafter named as reference D3), the European Patent EP-A1-0 679 378 (hereinafter named as reference D4), PCT international patent application. WO 96/11653 A (hereinafter named as reference D5), the German patent DE 100 11 687 A1 (hereinafter named as reference D6), respectively disclosed their own newly designed anti-snoring devices. Their common ground is that: 1. All were designed to be used on the upper jaw (wherein D3 could be used on the upper jaw and the lower jaw together); 2. Except that the fixer in D4 was designed to be a tooth plate, others were designed to be tooth covers that can encase the teeth; 3. All only were designed to have a so-called anti-snoring force-component acting on the big tongue, but either it was uncertain that which position of the big tongue (which is very important) was acted on by the force-component, or the force-component was designed at wrong positions.

The differences among D1-D6 are that: D1 and D2 mainly use screw and nut as the adjusting mechanism, coupled with the tongue-pressing pieces of different shapes; D4 uses a shaped hinge and a spring clip as the adjusting mechanism, coupled with a shaped bracket having a rotatable tongue-pressing loop; D6 uses the tension of a horizontal chain to adjust, coupled with a pressure pad with double link bars; while D3 only uses a closed semi-circlar loop as the tongue-pressing piece, D5 only uses a pikestaff-like strip body as the tongue-pressing piece. Evidently, these designs are either too complicated (such as D1, D2, D4 and D6), or too rigid (such as D3 and D5).

The first common feature of the above mentioned previous anti-snoring devices that these devices were designed to be used on the upper jaw is undoubtedly a right choice. Because if they were designed on the lower jaw, the reaction force exerted by the tongue to the tongue-pressing piece would have deviated from the fixed tooth cover or made the fixed tooth plate produce pain like pulling teeth through the clasp, so as to hamper the normal use of the anti-snoring devices. However, apart from the merit, the above mentioned previous anti-snoring devices have the following common shortcomings:
First, it is unfeasible to just have one force-component acting on the big tongue for anti-snoring. According to the applicant's long-term research, it has been demonstrated that the basic reason of snoring is the obstruction of the respiratory tract, specifically speaking, snoring is caused by the nasopharyngeal cavity blocked or partially blocked by the intra-oral organs (mainly big tongue base and uvula). The intra-oral structure of each people may be slowly deformed along with the increased age, increased fat and flabby muscle, etc. The palate and tongue form a natural barrier in the mouth to assist the food swallowing and air transferring. If the palate is gradually drooping and the big tongue is gradually raising, the inner form of mouth would be changed to result in blocking the tract, and form a "whistle" effect, the snoring and the suffocating are thus produced. Only restraining the uplifting of the big tongue (besides, such restrainability has a limit) without restraining the drooping of the soft palate simultaneously can not change the trend that the respiratory passage continues becoming narrower, thus can not really prevent snoring and suffocating.

Secondly, it may be because a large number of clinical trials were not carried out in the references D1-D6, so the researchers did not realize and can not yet disclose clearly that how to define the accurate position of the big tongue acted by the force-component, which is very crucial, and is related directly to whether the anti-snoring devices they designed is useful and effective or not. From their schematic figures, expect that the position can not be judged for the tongue showed in D1 is a retrusive tongue, the acting point in D3 and D4 is 1/2 of the tongue, i.e. the middle of the tongue; the acting point in D2, D5 and D6 is 3/3 of the tongue, i.e. the tongue base. However the applicant's further study has demonstrated that the force-component pressing the middle of the tongue not only causes the severe pain of the tongue, but also aggravated the uplift of the back of the tongue to cause more serious snoring; the force-component pressing the tongue base would cause nausea, and can not be tolerated by the majority of people, thus they can not be used to play the anti-snoring role.

Thirdly, since the known previous anti-snoring devices were either too complicated devices (such as D1, D2, D4 and D6), or too rigid (such as D3 and D5), they would have disadvantages if they are used in the oral clinical activities which need relatively great flexibility and have very limited space.

### CONTENTS OF THE INVENTION

The purpose of the present invention is to overcome the drawbacks of all kinds of prior anti-snoring devices, providing a new technical solution of a comfortable removable tongue position corrective anti-snoring device guaranteeing an unobstructed respiratory passage, which is concise and necessary, indeed makes patients easily adapt and accept it, and at the same time can indeed achieve the ideal anti-snoring and anti-suffocating effects.

The present invention is defined in claim 1. The technical scheme of the present invention is as follows: it makes use of wire or band material provided with suitable elasticity and rigidity to manufacture a comfortable removable tongue position corrective anti-snoring device whose fixer is mainly applied on upper jaw, characterized in that, it includes not only a nether force-component (3) connecting with the fixer (1), but also an upper force-component (2) connecting with the fixer (1), the force end of the said nether force-component (3) being designed to be suitable to the normal configuration of the posterior of the tongue, its head having a holding plate (4) and being designed to be located on the portion in front of the sensitive position of the posterior of the big tongue, and being able to press the big tongue downward/front downward without causing discomfort and nausea; the force end of the said upper force-component (2) being designed to be suitable to the normal configuration of the soft palate palatine, its head having a pressure plate (4) and being designed to be located on the portion in front of the sensitive position of the soft palate palatine, so as to suitably raise up the soft palate palatine and uvula (i.e."small tongue") upward/upward and backward without causing discomfort of peripheral tissue and nausea; the said upper and nether force-components (2) and (3) being made of suitable elastic and rigid material with a gap between them to enlarge and dredge the blocked respiratory tract, and on the premise of comfort, it works to prevent snoring and remit sleep apnoea syndrome (i.e. the suffocating).

### BRIEF DESCRIPTION OF THE APPENDED DRAWINGS

Next, a further description will be made as to the present invention with the specification figures.

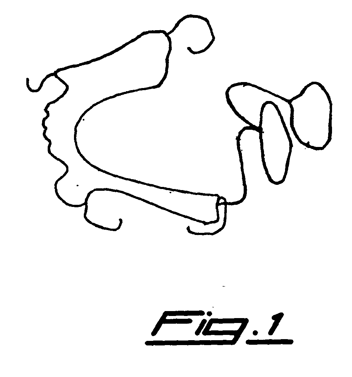

Fig.1 is an illustrative view showing the NiTi shape memory alloy anti-snoring device of Chinese utility model 90215295;
Fig.2 is an illustrative view showing an embodiment of the comfortable type removable tongue position corrective anti-snoring device according to the present invention;
Fig.3 is an illustrative view showing an example of a comfortable type removable tongue position corrective anti-snoring device ;
Fig.4 is an illustrative view showing a further example of a comfortable type removable tongue position corrective anti-snoring device according to the present invention;
Fig.5 is an illustrative view showing another example of a comfortable type removable tongue position corrective anti-snoring device ;
Fig.6 is an illustrative view showing another comfortable type removable tongue position corrective anti-snoring device ;
Fig.7 is an illustrative view showing a further comfortable type removable tongue position corrective anti-snoring device ;
Fig.8 is an illustrative view showing another example of a confortable type removable tongue position corrective anti-snoring device
Fig.9 is an illustrative view showing a further example of a comfortable type removable tongue position corrective anti-snoring device ;
Fig.10 is an illustrative view showing another comfortable type removable tongue position corrective anti-snoring device ;
Fig.11 is an illustrative view showing an embodiment of the comfortable type removable tongue position corrective anti-snoring device according to the present invention;
Fig.12 is an illustrative view showing examples and an embodiment of the comfortable type removable tongue position corrective anti-snoring device according to the present invention.

In the figures, 1 is the fixer, 2 is the upper force-component, 3 is the nether force-component, 4 is the holding plate/pressure plate, 5 is the base plate (tongue plate/palate plate/maxilla plate), 6 is the support bar (lingual bar/palatal bar/cheek bar/labial bar), 7 is the direct retainer (clasp/bent labial arch etc), 8 is the indirect retainer (hyoplastron/palate-plank/rest etc), 9 is the functional accessory (inclined bite plate/articulation plate/articulation mat etc), 10 is the circle retainer (band/dental crown cover etc), 11 is the link bar, 12 is the connecter, 13 is the adjustment, 14 is the teeth, 15 is the clip, 16 is the connecting wire, 17 is the group clips, 18 is the axial clip, 19 is the precision attachment/magnetic implant denture, 20 is the complete denture, 21 is the perdue false tooth, 22 is tooth cover.

### DESCRIPTION OF THE EMBODIMENTS

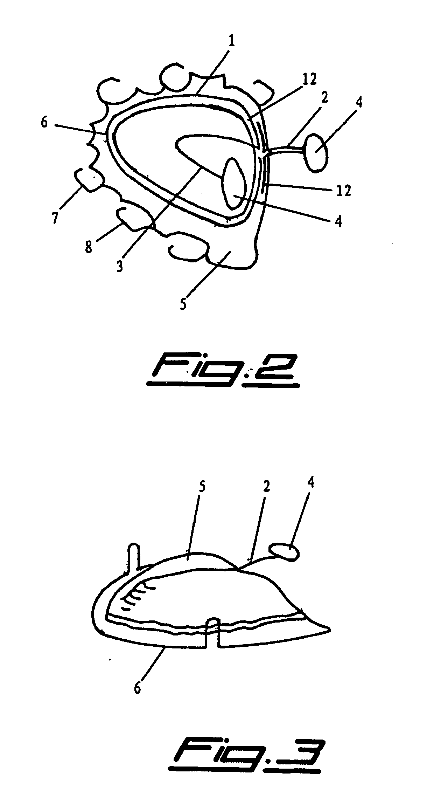

Referring to Fig.2, the comfortable type removable tongue position corrective anti-snoring device according to the present invention is designed to be applied on upper jaw, including fixer 1, upper force-component 2 and nether force-component 3, the upper force-component and nether force-component 3 are made of material (preferably high elastic alloy wire or band) provided with suitable elasticity and rigidity. The elongation part (i.e. the connecter 12) of the non-force end of the upper force-component 2 is embedded in the back area of the fixer 1, the other end i.e. force end extends backwardly and is designed to be suitable to the normal configuration of the soft palate palatine, the head is designed to be located on the portion in front of the sensitive position of the soft palate palatine and little withstand it. Thus, on the premise of not causing discomfort and nausea of peripheral tissue, it could bring an externally reinforced force to the soft palate palatine to prevent snoring. The connecter 12 of the non-force end of the nether force-component 3 is also embedded in the back area of the fixer 1, but the other end i.e. force end is normally bowed from middle lip side toward far end away the lip side like a bowed nose bridge along the palate, the bottom of bridge is designed to be suitable to the normal configuration of the posterior of the big tongue, the head is designed to be located on the portion in front of the sensitive position of the posterior of the big tongue, and press the big tongue downward/front downward, thus, on the premise of not causing discomfort and nausea of peripheral tissue, the tongue base is held effectively to prevent shrinking backward and press the palatine and uvula to cause the nasal cavity blocking and produce suffocating and snoring, and as mentioned above, the co-action of the upper and nether force-components 2, 3 could remarkably enlarge and keep the intra-oral effective inner space, thus could increase the breath efficiency and prevent developing sleep apnoea syndrome. The holding plate/pressure plate 4 set on the head portion of the force end of the upper and nether force-components 2, 3 is to buffer the force of the force-components 2, 3 and divide it onto the contact position evenly,. The holding plate/pressure plate 4 is generally made from synthetic resin (plastic etc) and/ or soft polymer material (silicon rubber etc), and its size should be suitable to the position.

In the comfortable type removable tongue position corrective anti-snoring device according to the present invention, the fixer 1 may have various forms. As Fig.2 shows, it is only a normal form, which is suitable for snoring patients with good teeth fixing. The fixer includes base plate 5 (here is a palate plate), support bar 6 (palatal bar) and direct retainer 7 (clasp etc), the whole anti-snoring device is fixed onto the suitable position of the upper jaw of the patient. And the indirect retainer 8 (palate-plank/rest etc) may be used in need to reinforce fixing and safety factor.

Referring to Fig.3, it shows a comfortable type removable tongue position corrective anti-snoring device including a fixer 1 having at least the base plate 5 and support bar 6 (here is a labial bar). It is designed to be applied on upper jaw, which includes not only the upper force-component 2, but also the nether force-component 3. The material and the making method of the upper force-component 2 and the nether force-component 3 are same to those in the example 1, but the fixer is slightly different. The fixer's base plate may be made of plastic with soft polymer material (silicon rubber etc) covering on it to increase the flexibility with the palate; In terms of need, the palate side may add the support bar 6 (palatal bar) to increase rigidity and/or decrease base plate's area; Vertical curve may be set on the labial bar for dismounting. This kind of the anti-snoring device is suitable for the snoring patient with good teeth layout of palate

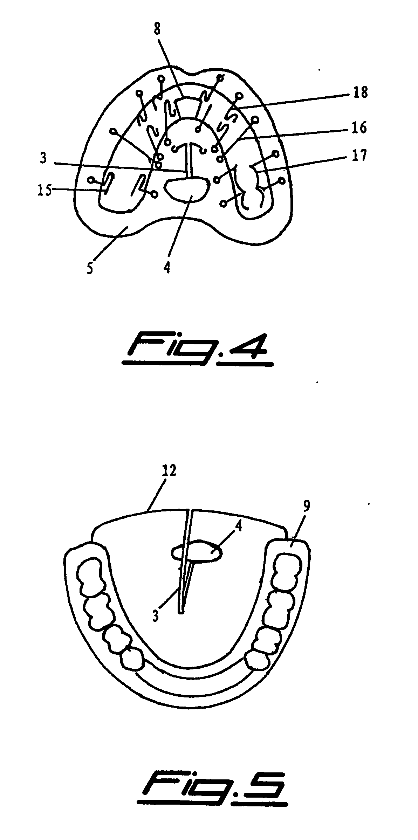

Referring to Fig.4, it shows a comfortable type removable tongue position corrective anti-snoring device , which includes at least base plate5 (here is a maxilla plate) and direct retainer7 (clip 15, connecting wire 16, group clips 17, axial clip 18 etc). It is designed to be applied on upper jaw, which includes not only the upper force-component 2, but also the nether force-component 3. The making method of the upper force-component 2 is also same to that in the example 1, but the force-component 3 is similar to a semi-tube with a seam in middle of it as figure shows The lip-cheek side and tongue-palate side of the maxilla plate may respectively make use of the partial support bar (labial bar/tongue palatal bar) to increase the rigidity and/or decrease base plate's area, an indirect retainer 8 (palate-plank/rest etc) may also be added to reinforce fixing, this kind of the anti-snoring device is suitable for the snoring patient with poor fixing.

Referring to Fig.5, it shows a comfortable type removable tongue position corrective anti-snoring device , which uses at least the functional accessory 9 (here is an articulation mat) as the fixer. It is designed to be applied on upper jaw, which includes not only the upper force-component 2, but also the nether force-component 3. The material and the making method of the upper force-component 2 and the nether force-component 3 are same to those in the example 1, but the fixer is slightly different. The fixer requires that the surface of the occlusal pad should have a deep groove to cover the tooth tip and corona. To increase rigidity, the support bar (tongue palatal bar/labial bar) may be set and the back area may be closed. This kind of the anti-snoring device is suitable for the snoring patient with good teeth fixing or small height.

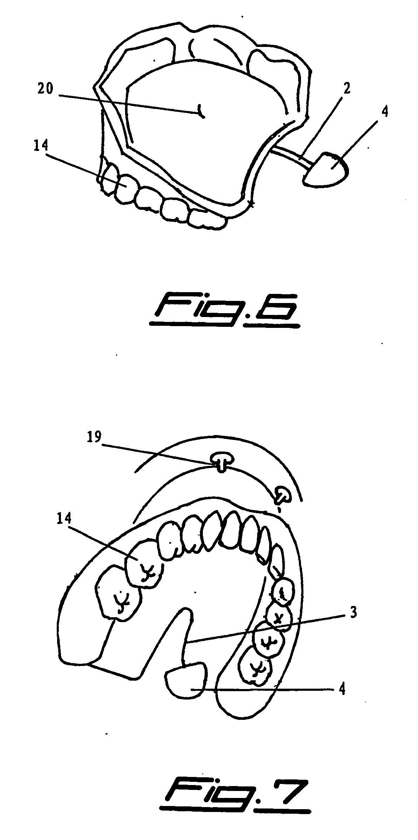

Referring to Fig.6, it shows a comfortable type removable tongue position corrective anti-snoring device which uses at least the complete denture as the fixer. It is designed to be applied on upper jaw, which includes not only the upper force-component 2, but also the nether force-component 3. The material and the making method of the upper force-component 2 and the nether force-component 3 are same to those in the example 1, but the fixer is slightly different. The tongue-palate side of the complete denture may also be added a support bar (tongue palatal bar) to decrease the base plate's area. This kind of the anti-snoring device is suitable for the snoring patient with non dental jaw.

Referring to Fig.7, it shows a comfortable type removable tongue position corrective anti-snoring device which uses at least the coving dental prosthesis or magnetic implant dental prosthesis as the fixer, is designed to be applied on upper jaw, and includes not only the upper force-component 2, but also the nether force-component 3. The material and the making method of the upper force-component 2 and the nether force-component 3 are same to those in the example 1, but the fixer is slightly different. The features of the fixing manner is that it has parts to form a complete set with gum precision attachment/button tract or magnetic implant denture 19. It is suitable for the snoring patient with bad teeth layout or high fixing requirement. Its back area may be closed, and added a support bar (tongue palatal bar etc) or netlike bracket to increase rigidity.

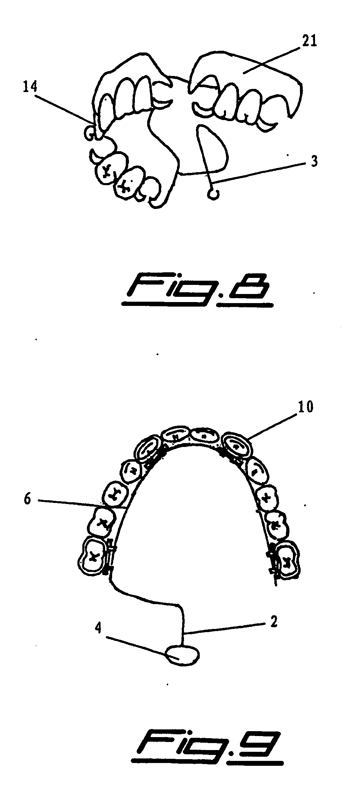

Referring to Fig.8, it shows a comfortable type removable tongue position corrective anti-snoring device which uses at least the perdue false tooth as the fixer. It is designed to be applied on upper jaw, which includes not only the upper force-component 2, but also the nether force-component 3. The material and the making method of the upper force-component 2 and the nether force-component 3 are same to those in the example 1, but the fixer is slightly different. A base plate (plastic) may be set on the tongue palate side of the concealed dental prosthesis to increase rigidity and embed the link bar (tongue palate bar etc), and its back area may be closed. This kind of the anti-snoring device is suitable for the snoring patient with non dental jaw and less teeth of high quality requirement.

Referring to Fig.9, it shows a comfortable type removable tongue position corrective anti-snoring device which uses at least the support bar 6 (here is a tongue palatal bar) and circle retainer 10 (band/dental crown cover etc) as the fixer. It is designed to be applied on upper jaw, which includes not only the upper force-component 2, but also the nether force-component 3. The material and the making method of the upper force-component 2 and the nether force-component 3 are same to those in the example 1, but the fixer is slightly different. The non-force end of the force-component is connected with the circle retainer 10 in a suitable way. This kind of the anti-snoring device is suitable for the snoring patient with good bow and teeth gap.

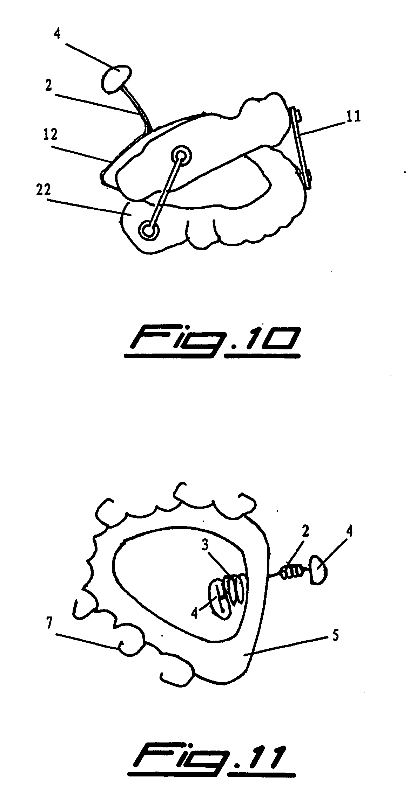

Referring to Fig.10, it shows a comfortable type removable tongue position corrective anti-snoring device which uses at least the tooth cover of the upper and lower jaws and cheek side support bar 11 as the fixer. It is designed to be applied on upper jaw, which includes not only the upper force-component 2, but also the nether force-component 3. The material and the making method of the upper force-component 2 and the nether force-component 3 are same to those in the example 1, but the fixer is slightly different. A base plate (plastic) may be set on the tongue palate side of the tooth cover, and the support bar (tongue palatal bar) may be embeded in the plastic base plate, and the back area can be reinforced. This kind of the anti-snoring device is suitable for the snoring patient with the shrinking backward of the lower jaw and abnormal tongue position.

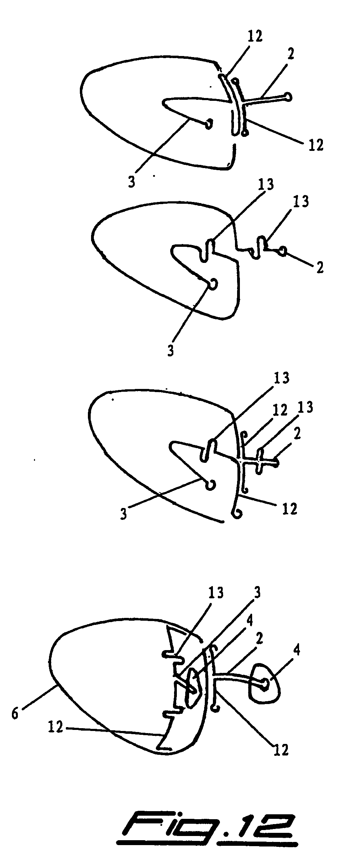

It should be noticed that the upper force-component 2 and nether force-component 3 are the most special parts of the anti-snoring device, enough elasticity and suitable rigidity are both extremely important, any one of which is indispensable. Meanwhile, the suitable thickness or diameter is important also, it may directly affect the elasticity. In design, the force-component piece 2, 3 may be connected with the support bar 6, or separated as Fig.12 shows; it may use same material and size or different material and size. The force end of the force-component 3 may point directly to the end position in form of spring, single wire and band or spring tube (as Fig.11 and Fig.4 show), or indirectly to the end position (as Fig.5 and Fig.8 show); The middle of the force-component 2, 3 may at least have an adjustment 13 to adjust the length and angle as Fig.12 shows. Meanwhile, the support bar 6 may be a lingual bar, palatal bar, cheek bar, labial bar etc; the direct retainer 7 may be a clasp and bendted labial arch etc; the indirect retainer 8 may be a hyoplastron, palate-plank, rest etc; the functional accessory 9 may be a inclined bite plate, articulation plate or articulation mat etc.

The comfortable and type removable tongue position corrective anti-snoring device above, proved by about one thousand clinical trials, is concise, reasonable and convenient, and has a good clinic effect with a better design result, it has wider applicable range with a better effect for more than 98% patients, thus it can substitute the ventilator without any side effects.

## Claims

1. A comfortable type removable tongue position corrective anti-snoring device, comprising:
a fixer (1);
a nether force-component (3) connecting with the fixer (1); and
an upper force-component (2) connecting with the fixer (1);
wherein the fixer is mainly located at the upper jaw, but can also be located at the upper jaw and the lower jaw together,
the force end of said nether force-component (3) being designed to be suitable to the normal configuration of the posterior of the tongue, its head having a holding plate (4) and being designed to be located on the portion in front of the sensitive position of the posterior of the big tongue, thus being able to press the big tongue downward/frontward and downward without causing discomfort of peripheral tissue and nausea;
the upper and nether force-components (2) and (3) being made of suitable elastic and rigid material with a gap between them to enlarge the intra-oral inner space, i.e. intra-oral tract, to increase the efficiency of breath and play the anti-snoring and anti-suffocating role,
**characterised in that** the force end of said upper force-component (2) is designed to be suitable to the normal configuration of the soft palate palatine velum, its head having a pressure plate (4) and being designed to be located on the portion in front of the sensitive position of the soft palate palatine velum, so as to suitably raise up the soft palate palatine velum and uvula, i.e. "small tongue", upward/upward and backward without causing discomfort of peripheral tissue and nausea.

2. The comfortable and type removable tongue position corrective anti-snoring device according to Claim 1, wherein the non-force end of the said upper force-component (2) is connected with the postdam area of the fixer (1), then bows along the palatine median suture to the anterior lip, the non-force end of the said nether force-component (3) being also connected with the postdam area of the fixer (1), then bowing first along the palatine median suture to the anterior lip, then bowing downwardly and backwardly away from the anterior lip, like a nose-like protuberance having at least one bend seen from the lip side, there being an appropriate space between the upside and the bottom of the nose-like protuberance.

3. The comfortable type removable tongue position corrective anti-snoring device according to Claim 1or 2, wherein the said upper force-component (2) and/or nether force-component (3) are at least made of wire or band material, the wire or band material having suitable thickness or diameter.

4. The comfortable type removable tongue position corrective anti-snoring device according to any one of Claims 1 to 3, wherein the said upper force-component (2) and/or nether force-component (3) are made of elastic wire and/or band.

5. The comfortable type removable tongue position corrective anti-snoring device according to any one of Claims 1 to 4, wherein the holding plate/pressure plate (4) has a suitable shape and size and may be made from synthetic resin and/or soft polymer material.

6. The comfortable type removable tongue position corrective anti-snoring device according to Claim 1, wherein the said fixer (1) is made up of at least any one of the base plate (5), support bar (6), direct retainer (7), indirect retainer (8), functional accessory (9); its material being at least any one of synthetic resin, metal, soft polymer material, concealed dental prosthesis material.

7. The comfortable type removable tongue position corrective anti-snoring device according to Claim 1, wherein the said fixer may be made of covering dental prosthesis or tooth cover or magnetic implant dental prosthesis, or at least made of support bar (6) and circle retainer (10) connected with support bar (6).

8. The comfortable type removable tongue position corrective anti-snoring device according to Claim 1, wherein each cheek side has a link bar (11) which could work to move the jaws in case of setting the said fixer (1) on the upper and lower jaws simultaneously.

9. The comfortable type removable tongue position corrective anti-snoring device according to Claims 1 and 4, wherein the elongation part, i.e. connector (12), of the non-force end of the upper and nether force-components (2, 3) is connected with the support bar (6), or separated; it may use same material and size, or different material and size.

10. The comfortable type removable tongue position corrective anti-snoring device according to Claims 1 and 2, wherein the middle portions of the said upper and nether force-components (2, 3) at least have an adjustment (13) to adjust the length and position in clinical use.

## Patentansprüche

1. Eine bequeme, entfernbare, die Zungenposition korrigierende Antischnarchvorrichtung umfassend:
- ein Fixierteil (1),
- eine untere Wirkungskomponente (3), die mit dem Fixierteil (1) verbunden ist, und
- eine obere Wirkungskomponente (2) die mit dem Fixierteil (1) verbunden ist,
wobei das Fixierteil hauptsächlich am Oberkiefer anliegt, aber auch am Oberkiefer und am Unterkiefer gemeinsam anliegen kann, und das wirksame Ende der besagten unteren Wirkungskomponente (3) so gestaltet ist, dass es für die normale Konfiguration des hinteren Teils der Zunge geeignet ist und sein Kopf eine Halteplatte (4) aufweist und dafür vorgesehen ist, auf dem Abschnitt vor der empfindlichen Position des hinteren Teils der großen Zunge angeordnet zu sein, wodurch er in der Lage ist, die große Zunge nach unten bzw. nach vorne unten zu drücken, ohne Beschwerden des umgebenden Gewebes und Übelkeit zu verursachen, wobei die obere und untere Wirkungskomponente (2) bzw. (3) aus einem geeigneten elastischen und starren Material bestehen und zwischen ihnen ein Abstand vorgesehen ist, um den Innenraum der Mundhöhle zu vergrößern, damit die Effizienz des Atmens verbessert und Schnarchen und Ersticken verhindert wird, **dadurch gekennzeichnet, dass** das wirksame Ende der besagten oberen Wirkungskomponente (2) so gestaltet ist, dass es für die normale Konfiguration des Gaumensegels (Velum) geeignet ist und sein Kopf eine Halteplatte (4) aufweist und dafür vorgesehen ist, auf dem Abschnitt vor der empfindlichen Position des Gaumensegels (Velum) angeordnet zu sein, so dass er auf geeignete Weise das Gaumensegel (Velum) und das Zäpfchen, d. h. die "kleine Zunge", nach oben bzw. oben hinten hebt, ohne Beschwerden des umgebenden Gewebes und Übelkeit zu verursachen.

2. Die bequeme, entfernbare, die Zungenposition korrigierende Antischnarchvorrichtung gemäß Anspruch 1, bei der das nicht wirksame Ende der besagten oberen Wirkungskomponente (2) mit dem hinteren Abdämmgebiet des Fixierteils (1) verbunden ist und dann einen Bogen entlang der medianen Gaumennaht zur vorderen Lippe beschreibt, und bei der das nicht wirksame Ende der besagten unteren Wirkungskomponente (3) ebenfalls mit dem hinteren Abdämmgebiet des Fixierteils (1) verbunden ist und zuerst einen Bogen entlang der medianen Gaumennaht zur vorderen Lippe beschreibt und dann einen Bogen nach unten und hinten von der vorderen Lippe weg beschreibt, wie ein nasenförmiger Höcker mit von der Lippenseite gesehen mindestens einer Biegung, wobei ein geeigneter Abstand zwischen der Oberseite und dem unteren Teil des nasenförmigen Hockers vorgesehen ist.

3. Die bequeme, entfernbare, die Zungenposition korrigierende Antischnarchvorrichtung gemäß Anspruch 1 oder 2, bei der besagte obere Wirkungskomponente (2) und/oder untere Wirkungskomponente (3) mindestens aus einem Draht- oder Bandmaterial bestehen bzw. besteht, das eine geeignete Dicke oder einen geeigneten Durchmesser aufweist.

4. Die bequeme, entfernbare, die Zungenposition korrigierende Antischnarchvorrichtung gemäß einem der Ansprüche 1 bis 3, bei der besagte obere Wirkungskomponente (2) und/oder untere Wirkungskomponente (3) aus einem elastischen Draht und/oder Band bestehen bzw. besteht.

5. Die bequeme, entfernbare, die Zungenposition korrigierende Antischnarchvorrichtung gemäß einem der Ansprüche 1 bis 4, bei der die Halteplatte/Druckplatte (4) eine geeignete Form und Größe hat und aus einem Kunstharzmaterial und/oder einem Weichpolymermaterial bestehen kann.

6. Die bequeme, entfernbare, die Zungenposition korrigierende Antischnarchvorrichtung gemäß Anspruch 1, bei der das besagte Fixierteil (1) mindestens eine der Komponenten Grundplatte (5), Stützbügel (6), direkter Haltebügel (7), indirekter Haltebügel (8), funktionelles Zubehör (9) umfasst und mindestens aus einem der folgenden Materialien besteht: Kunstharz, Metall, Weichpolymer, Material verdeckter Zahnprothesen.

7. Die bequeme, entfernbare, die Zungenposition korrigierende Antischnarchvorrichtung gemäß Anspruch 1, bei der besagtes Fixierteil aus einer abdeckenden Zahnprothese oder einer Zahndecke oder einer Magnetimplantat-Zahnprothese besteht oder mindestens aus einem Stützbügel (6) und einem mit dem Stützbügel (6) verbundenen Kreisbügel (10) besteht.

8. Die bequeme, entfernbare, die Zungenposition korrigierende Antischnarchvorrichtung gemäß Anspruch 1, bei der jede Backenseite einen Verbindungsbügel (11) aufweist, der im Gebrauch die Kiefer bewegen könnte, falls besagtes Fixierteil (1) gleichzeitig auf den Oberkiefer und den Unterkiefer aufgesetzt wird.

9. Die bequeme, entfernbare, die Zungenposition korrigierende Antischnarchvorrichtung gemäß Ansprüchen 1 und 4, bei der das Verlängerungsteil, d. h. der Verbinder (12), des nicht wirksamen Endes der oberen und der unteren Wirkungskomponente (2, 3) mit dem Stützbügel (6) verbunden ist oder davon getrennt ist; es kann aus dem gleichen Material bestehen und von gleicher Größe sein oder aus einem anderen Material bestehen und von anderer Größe sein.

10. Die bequeme, entfernbare, die Zungenposition korrigierende Antischnarchvorrichtung gemäß Ansprüchen 1 und 2, bei der die Mittelabschnitte der besagten oberen und unteren Wirkungskomponenten (2, 3) mindestens eine Verstellung (13) der Länge und der Position im klinischen Einsatz umfassen.

## Revendications

1. Dispositif confortable anti-ronflement avec correction de la position de la langue, de type amovible, comprenant :
un dispositif de fixation (1) ;
un composant de force inférieur (3) se raccordant avec le dispositif de fixation (1) ; et
un composant de force supérieur (2) se raccordant avec le dispositif de fixation (1) ;
dans lequel le dispositif de fixation est principalement positionné au niveau de la mâchoire supérieure, mais peut également être positionné au niveau de la mâchoire supérieure et de la mâchoire inférieure,
l'extrémité de force dudit composant de force inférieur (3) étant conçue pour être appropriée à la configuration normale de l'arrière de la langue, sa tête ayant une plaque de support (4) et étant conçue pour être positionnée sur la partie située en face de la position sensible de l'arrière de la langue, tout en pouvant comprimer la partie postérieure de la langue vers le bas/l'avant et le bas sans provoquer d'inconfort du tissu périphérique ni de nausée ;
les composants de force supérieur et inférieur (2) et (3) étant réalisés avec une matière appropriée élastique et rigide avec un espace entre eux pour agrandir l'espace interne intrabuccal, c'est-à-dire le tractus intrabuccal afin d'augmenter l'efficacité de la respiration et jouer le rôle anti-ronflement et anti-suffocation,
**caractérisé en ce que** l'extrémité de force dudit composant de force supérieur (2) est conçue pour être appropriée à la configuration normale du voile du palais souple, sa tête ayant une plaque de pression (4) et étant conçue pour être positionnée sur la partie en face de la position sensible du voile du palais souple afin de faire remonter de manière appropriée le voile du palais souple et l'uvule, c'est-à-dire « la partie avant de la langue » vers le haut/vers le haut et vers l'arrière sans provoquer d'inconfort du tissu périphérique ni de nausée.

2. Dispositif anti-ronflement confortable avec correction de la position de la langue, de type amovible selon la revendication 1, dans lequel l'extrémité sans force dudit composant de force supérieur (2) est raccordée avec la zone arrière du dispositif de fixation (1), s'arque ensuite le long de la suture médiane du palais vers la lèvre antérieure, l'extrémité sans force dudit composant de force inférieur (3) étant également raccordée avec la zone arrière du dispositif de fixation (1), s'arquant ensuite tout d'abord le long de la suture médiane du palais vers la lèvre antérieure, s'arquant ensuite vers le bas et l'arrière à distance de la lèvre antérieure, comme une protubérance en forme de nez ayant au moins un fléchissement observé depuis le côté de la lèvre, où se trouve un espace approprié entre le côté supérieur et le fond de la protubérance en forme de nez.

3. Dispositif confortable anti-ronflement avec correction de la position de la langue, de type amovible selon la revendication 1 ou 2, dans lequel ledit composant de force supérieur (2) et/ou le composant de force inférieur (3) sont au moins réalisés avec un matériau en fil ou en bande, le matériau en fil ou en bande ayant une épaisseur ou un diamètre approprié(e).

4. Dispositif confortable anti-ronflement avec correction de la position de la langue, de type amovible selon l'une quelconque des revendications 1 à 3, dans lequel le composant de force supérieur (2) et/ou le composant de force inférieur (3) sont réalisés avec un fil et/ou une bande élastique.

5. Dispositif confortable anti-ronflement avec correction de la position de la langue, de type amovible selon l'une quelconque des revendications 1 à 4, dans lequel la plaque de support/plaque de pression (4) a une forme et une dimension appropriées et peut être réalisée à partir de résine synthétique et/ou d'une matière polymère souple.

6. Dispositif confortable anti-ronflement avec correction de la position de la langue, de type amovible selon la revendication 1, dans lequel ledit dispositif de fixation (1) est composé d'au moins l'un quelconque parmi la plaque de base (5), la barre de support (6), un dispositif de retenue direct (7), un dispositif de retenue indirect (8), un accessoire fonctionnel (9) ; son matériau étant au moins l'un quelconque parmi une résine synthétique, un métal, une matière polymère souple, une matière de prothèse dentaire dissimulée.

7. Dispositif confortable anti-ronflement avec correction de la position de la langue de type amovible selon la revendication 1, dans lequel ledit dispositif de fixation peut être réalisé avec une prothèse dentaire de recouvrement ou un recouvrement de dent ou une prothèse dentaire à implant magnétique ou au moins réalisé avec la barre de support (6) et un dispositif de retenue circulaire (10) raccordé avec la barre de support (6).

8. Dispositif confortable anti-ronflement avec correction de la position de la langue, de type amovible selon la revendication 1, dans lequel chaque côté de joue a une barre de liaison (11) qui peut travailler pour déplacer les mâchoires en cas de placement dudit dispositif de fixation (1) sur les mâchoires supérieure et inférieure simultanément.

9. Dispositif confortable anti-ronflement avec correction de la position de la langue, de type amovible selon les revendications 1 et 4, dans lequel la partie d'allongement, c'est-à-dire le connecteur (12) de l'extrémité sans force des composants de force supérieur et inférieur (2, 3) est raccordé avec la barre de support (6) ou séparé ; il peut utiliser le même matériau et la même taille ou un matériau différent et une taille différente.

10. Dispositif confortable anti-ronflement avec correction de la position de la langue, de type amovible selon les revendications 1 et 2, dans lequel les parties centrales desdits composants de force supérieur et inférieur (2, 3) ont au moins un ajustement (13) pour ajuster la longueur et la position, à l'usage clinique.
